# EUROPEAN PATENT APPLICATION

(11) **EP 1 417 944 A1**
(43) Date of publication of application: **12.05.2004**
(21) Application number: 03103738.5
(22) Date of filing: 09.10.2003
(51) Int. Cl.: A61F 11/14, H04R 1/10, A42B 3/16, A61F 9/02

(54) **Cushions for covering a wearer's body**

(30) Priority: 14.10.2002 GB 0223894
(71) Applicant: Thales Plc, Fleet, Hampshire GU51 3NY (GB)
(72) Inventor: COZENS, Ray, 94117, ARCUEIL (FR); HILL, Neal, 94117, ARCUEIL (FR); LINSCOTT, Peter, 94117, ARCUEIL (FR); MOULTON, Dave, 94117, ARCUEIL (FR)
(74) Representative: Lucas, Laurent Jacques

(57) **Abstract**

There is disclosed apparatus (10) for covering some or all of a wearer's body, including interface means (4) which in use are adjacent the wearer's body. The interface means including a first region of material (8) having a first compliance (A) and a second region of material (10) having a second compliance (B), the first compliance being greater than the second compliance. Both of the regions are adjacent respective parts of the wearer's body.

## Description

The present invention relates to cushions and in particular to the types of cushions used on apparatus which is intended to be worn on the head of a user, for example ear defenders, headphones, masks, goggles and close fitting helmets.

In prior art headphones and ear defenders, each earpiece normally includes a rigid ear cup (which fits over an ear of the wearer) together with some cushioning material fixed around the perimeter of the ear cup to make the ear cup more comfortable to wear, to provide some level of acoustic seal, and provide some level of attenuation (in low frequency environments). One problem is that a relatively soft cushioning material, when combined with an ear cup provides a relatively high degree of comfort but poor acoustic sealing and attenuation. Likewise, relatively hard cushioning material, when combined with an ear cup provides good acoustic sealing and attenuation but low comfort.
'Acoustic Seal' may be defined as zero leak paths between the cushion and body of the user.
'Acoustic Attenuation' may be defined as the mean difference in decibels between the threshold of hearing with and without the hearing pr otector in place. (BS EN 352-1:1993 Hearing Protector, Part 1)

United States Patent No. 4856118 describes a headphone cushion which includes two concentric rings on the perimeter of each ear cup. These concentric rings are made of a non-liquid gelatine-like silicone and are mounted on a layer of slow recovery foam enclosed in a thin stretchable layer of polyurethane skin. The aim is stated to be to provide a good acoustic seal between a headphone cover and the head and ear of a wearer while being relatively lightweight and comfortable to wear. However, as explained above, with the prior art designs these two parameters are to some extent mutually exclusive and so the present invention aims to provide a design which improves on this.

Accordingly, in a first aspect, the present invention provides apparatus for covering some or all of a wearer's body, including interface means which in use are adjacent the wearer's body, the interface means including a first region of material having a first compliance and a second region of material having a second compliance, the first compliance being greater than the second compliance, wherein both of the regions are adjacent respective parts of the wearer's body.

The use of two different regions of material having respectively different compliances enables the interface means to be designed so as to provide a better seal between the remainder of the apparatus and the relevant part of the wearer's body which the apparatus covers. In some practical examples, such as goggles, it is merely the "tightness" of the seal which is important and the present invention enables a suitably tight seal to be achieved at an increased level of comfort in comparison to the prior art. Additionally or alternatively, in examples where the acoustic attenuation of the cushion is important (for example headsets, ear defenders or headphones), for a given level of comfort the present invention enables an enhanced acoustic attenuation in comparison to the prior art

Preferably the part of the wearer's body on which the apparatus is worn is the head and in some examples may be the face region (e.g. a face mask), the eye region (e.g. goggles), the ear region (e.g. headsets, ear defenders, headphones) or the cranial region (e.g. helmets).

Usually the apparatus will have an area, often a perimeter, designed or shaped to fit the relevant part of the wearer's body. Generally, the interface means will be attached to this area or perimeter and preferably to the whole area or perimeter, so that, in some examples, the interface means is the only part of the apparatus to be adjacent or contact the relevant part of the wearer's body. Preferably the interface means covers the whole of the area or perimeter and more preferably the second region of the interface means does not cover the whole area or perimeter but only a part of it. The first region of the interface means may cover the whole area or perimeter or alternatively it may cover only a part of it also.

In some practical examples, the first region may cover substantially all of the area of perimeter and have sections where it is effectively replaced by the second region of material. The arrangement may be such that the first region wholly or partly encloses the second region - for example, on the side of the interface means which is closest to the relevant part of the wearer's body in use, the second region may be as close to the wearer's body as the first region or may be covered by the first region.

Either or both of the regions may be covered with a suitable protective material e.g. may be skinned or integrally skinned. Alternatively, either or both of the regions may be uncovered.

In practice, the apparatus may be designed so that in use the first region is adjacent sensitive areas of the wearer's body (e.g. where arteries, veins and/or nerves are located near the surface) and the second region located adjacent less sensitive areas (e.g. where bone is close to the surface).

In a further aspect, the present invention provides a method of constructing apparatus for covering all or a part of a wearer's body, the apparatus including interface means which in use are adjacent the wearer's body, the method including providing a first region of material of a first compliance and the second region of material of a second compliance, the first compliance being greater than the second compliance wherein both regions are adjacent respective parts of the wearer's body in use.

Embodiments of the present invention will now be described with reference to the accompanying drawings in which:
Fig. 1 is a perspective view of an embodiment of an ear cup including a cushion according to the present invention;
Fig. 2 is a cross-sectional view along the line A-A in Fig. 1;
Fig. 3 is a sectional perspective view of part of an interface member according to an embodiment of the present invention;
Fig. 4 is a cross-sectional view along the line X-X in Fig. 3;
Fig. 5 is an alternative possibility for the cross-sectional view X-X in a different embodiment of an interface member according to the present invention;
Fig. 6 is a further alternative possibility for the cross-sectional view X-X in a different embodiment of an interface member according to the present invention;
Fig. 7 is a cross-sectional view along the line Z-Z in Fig. 3;
Fig. 8 is an alternative possibility for the cross-sectional view Z-Z in a different embodiment of an interface member according to the present invention;
Fig. 9 is a further alternative possibility for the cross-sectional view Z-Z in a different embodiment of an interface member according to the present invention.
Fig.10 is an exploded sectional perspective view showing a construction of independent materials.

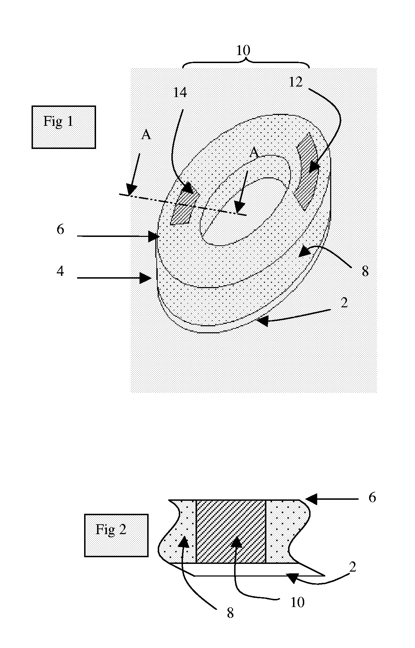

Figs. 1 and 2 will be described with reference to an embodiment of the present invention which is an ear cup including a cushion (e.g. for an acoustic noise attenuating headset, ear defenders or headphones etc) but the concept may equally be applied to interface members for other types of apparatus as explained above, for example face masks or goggles or close fitting helmets etc. Similarly, Figs. 3 - 9 show some possible configurations of such interface members and those configurations may be substituted in the ear cushion of Fig. 1 as well as being suitable for use in the other types of apparatus envisaged in this application.

In Fig.1, the ear cushion may include a substrate or backing plate 2 on which is mounted a cushioning member 4 (referred to above generally as interface means). In this case, the cushioning member is generally rectangular in cross-section (preferably with radiused corners) and oval in shape so as to conform to the perimeter of the substrate 2. An upper surface 6 of the cushioning member is intended to lie adjacent the head of the wearer in use.

The cushioning member 4 includes two regions of material, the first region 8 has a first compliance A and the second region 10 has a second compliance B, where compliance B is less than compliance A. In fact, in this example the region 10 is split into two parts 12 and 14. In use, part 12 is intended to lie adjacent the skull bone behind the upper area of a wearer's ear and part 14 is intended to lie adjacent the temporal process.

The aim of this embodiment of the present invention is to provide material of relatively high comfort (i.e. relatively high compliance or low density) adjacent or against those areas of the wearer's ear region (pinna) which are sensitive (such as the temple area and the area at the base of the ear), so the apparatus is comfortable to wear. In addition, areas of relatively low compliance (i.e. high stiffness or density) are provided so as to lie adjacent or press against relatively less sensitive areas of the wearer's ear region. This enables the cushion as a whole to provide a relatively more comfortable seal and, in the case of a hearing protector, better acoustic attenuation where it is useful.

Where the level of acoustic attenuation is significant, the attenuation is a function of the stiffness K of any given material (e.g. ear cushion material). Stiffness K is the inverse of the compliance C of a material i.e. C = 1/K. In the above example, the stiffness of the second region 10 of material dominates the system under load (i.e. in use) and therefore the attenuation provided by the apparatus is largely determined by the stiffer region 10, whilst still providing levels of comfort according to region 8.

Fig. 2 is a cross-sectional view along the line A-A of Fig. 1 through region 14. Region 12 may have a similar cross-sectional view or alternatively may be different. Cross-sectional views of alternative embodiments could be as shown in any of Figs. 4 - 9, for example.

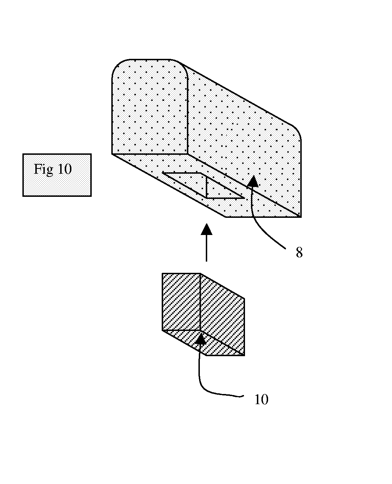

In Fig. 2 it can be seen that effectively a section of material 8 has either been removed or not manufactured in the first place and in its place is located the region 10 of the second material (Ref Fig. 10). The upper surface 6 of the ear cushion is intended to press against the wearer's head in use and, as can be seen, both regions 8 and 10 will press against respective parts of the head of the wearer.

However, as shown in Figs. 3 - 9, the upper surface of the cushion may also include a skin or cover which may be applied separately or fabricated as part of the materials for regions 8 and/or 10. Furthermore, in the alternative embodiments illustrated by Figs. 4 and 6, it can be seen that the region 10 does not directly contact the body of the wearer (or the cover or skin of the apparatus, where appropriate) but instead is itself wholly or partly covered by some of the region 8.

Polyurethane Foam (possibly with a skin may be used as suitable cushion material.

Fig. 3 shows a sectional view through part of a cushion or interface means according to an embodiment of the present invention. As stated above, such a cushion could be used as part of, for example, an ear defender according to Fig.1 or could be used as the cushioning member for many other types of apparatus as previously described.

In Fig. 3, the cushioning member (generally 30) may sits on a base or substrate 32. The cushioning member includes an optional cover or skin 34 which covers the cushioning material. As in the embodiment of Fig. 1, the cushioning material includes two regions and the same numbering will be used for clarity - region 8 is a first region of relatively high compliance material and region 10 is a second region of relatively low compliance material.

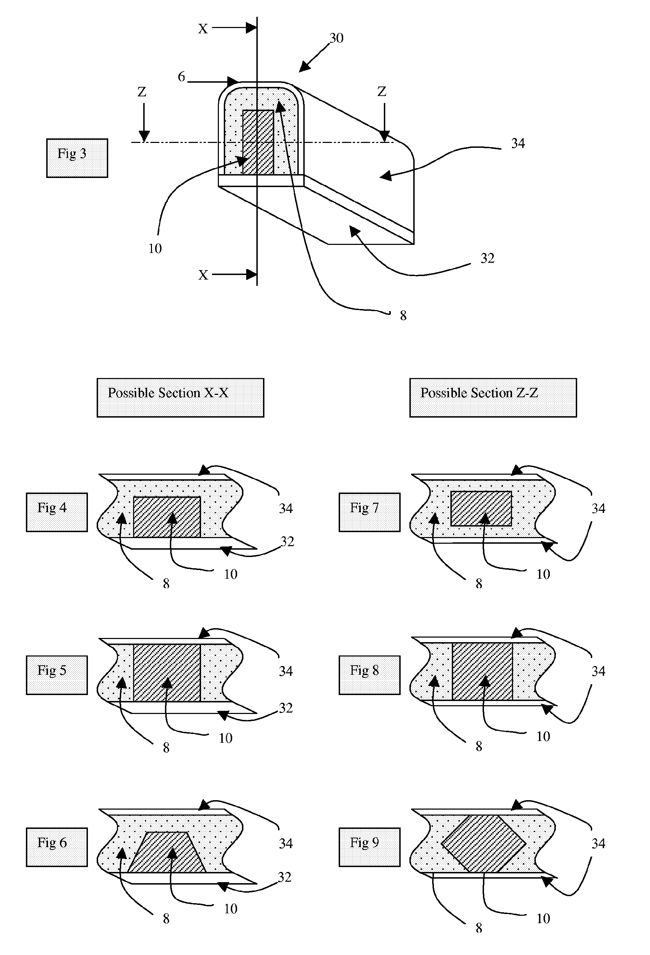

In this example, the first region of material 8 encloses the region of material 10 on all sides other than that which contacts the substrate 32. This is clear from the cross-sectional view shown in Figs. 4 and 7. However, alternative embodiments are possible and examples of these are shown in the cross-sectional view of Figs. 5,6,8 & 9. For example, the region material 10 may extend from the substrate 32 to the upper surface 6, or only part of the way. Additionally or alternatively, the region 10 may extend from one side of the cushion member to the other or only part of the way.

Fig. 10 shows an exploded view of a cushion member similar to that of Fig. 3. In Fig. 10, the cushion member is constructed in two parts - a region of material 10 is inserted into an appropriate space in the first region of material 8.

In this and other embodiments (including the embodiment of Fig. 1) the substrate need not be an independent item but instead could simply be a portion of the apparatus to which the cushioning member is attached e.g. the ear cup in the embodiment of Fig. 1.

The above embodiments are intended to be an example of the present invention and variants and modifications of those embodiments, such as would be readily apparent to the skilled person, are envisaged and may be made without departing from the scope of the present invention. For example, the above embodiments have been described with reference only to two regions of material of respectively different compliances, but it is possible to include further regions of material having other compliance levels as appropriate for the particular article to which the cushion is applied.

## Claims

1. Apparatus for covering some or all of a wearer's body, including interface means which in use are adjacent the wearer's body, the interface means including a first region of material having a first compliance and a second region of material having a second compliance, the first compliance being greater than the second compliance, wherein both of the regions are adjacent respective parts of the wearer's body.

2. Apparatus according to Claim 1 wherein the part of the wearer's body on which the apparatus is worn is the head, the face region, the eye region, the ear region or the cranial region.

3. Apparatus according to Claim 1 or Claim 2 wherein it is arranged so that in use the first region is adjacent sensitive areas of the wearer's body and the second region located adjacent less sensitive areas.

4. Apparatus according to any claim wherein the apparatus has an area, designed or shaped to fit the relevant part of the wearer's body and the interface means is attached to this area so that, the interface means contacts the relevant part of the wearer's body in use.

5. Apparatus according to Claim 4 wherein the interface means covers the whole of the area and the second region of the interface means does not cover the whole area or perimeter but only a part of it.

6. Apparatus according to Claim 5 wherein the interface means covers the whole area or alternatively covers the whole area.

7. Apparatus according to Claim 5 or Claim 6 wherein the first region wholly or partly encloses the second region.

8. Apparatus according to any of the above claims wherein either or both of the regions is covered with a suitable protective material.

9. Apparatus according to any of the above claims wherein the apparatus is an ear cup to be worn over the ear of a wearer, and arranged such that in use the first region lies adjacent the temple of the wearer and the second region lies adjacent the top of the ear of the wearer.

10. A method of constructing apparatus for covering all or a part of a wearer's body, the apparatus including interface means which in use are adjacent the wearer's body, the method including providing a first region of material of a first compliance and the second region of material of a second compliance, the first compliance being greater than the second compliance wherein both regions are adjacent respective parts of the wearer's body in use.
